# EUROPÄISCHE PATENTSCHRIFT

(11) **EP 0 775 481 B1**
(45) Veröffentlichungstag und Bekanntmachung des Hinweises auf die Patenterteilung: **12.06.2002**
(21) Anmeldenummer: 96117142.8
(22) Anmeldetag: 25.10.1996
(51) Int. Cl.: A61K 7/48, A61K 7/027, A61K 7/025

(54) **Stabile kohlenwasserstofffreie kosmetische oder dermatologische Oleogele und O/W-Emulsionen**
Stable and free of hydrocarbons cosmetic or dermatologic oleogels and O/W emulsions
Oléogels et émulsions R/E cosmetiques ou dermatologiques stables et exempts d'hydrocarbones

(30) Priorität: 10.11.1995 DE 19541968
(43) Veröffentlichungstag der Anmeldung: 28.05.1997
(73) Patentinhaber: Beiersdorf Aktiengesellschaft, 20245 Hamburg (DE)
(72) Erfinder: Meiring, Uta, 20251 Hamburg (DE); Dussert, Anne-Sopie, 31400 Toulouse (FR); Aul, Arta, 21244 Buchholz (DE)

(56) Entgegenhaltungen:
- WO-A-93/08840
- FR-A- 2 290 414
- US-A- 4 431 673
- US-A- 4 639 369

## Beschreibung

Die vorliegende Erfindung betrifft stabile kohlenwasserstofffreie kosmetische oder dermatologische Formulierungen in Form von Oleogelen und W/O-Emulsionen. Insbesondere betrifft die vorliegende Erfindung solche Zubereitung zum Zwecke der Lippenpflege.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen. (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die Haut der Lippen im besonderen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trokkenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegeformulierungen, welche meistens in Form von Lippenpflegestiften erhältlich sind. Diese Produkte enthalten meist zu einem hohen Anteil Wachse und Fettkomponenten, die nach dem Auftragen eine abdeckende Schicht über den Lippen ausbilden. Weniger gebräuchlich, aber nicht unüblich sind Zubereitungen in flüssiger oder halbfester Form, beispielsweise Lippenpflegegelen oder - Cremes.

Die Lederhaut der Lippen weist gut durchblutete Papillen auf, die bis dicht unter die Lippenoberfläche reichen. Daher sind die Lippen rötlich gefärbt und, je nach Teintfarbe der betreffenden Person, von der übrigen Gesichtshaut mehr oder weniger stark farblich abgesetzt. Ein Stilmittel der dekorativen Kosmetik ist dann auch, die Lippenfarbe durch entsprechende Kosmetika auf den Typ der Person abzustimmen.

Produkte dieser Art sind beispielsweise dekorative Lippenstifte, in welche verschiedenste Farbpigmente eingearbeitet werden können. Auch diese Stifte enthalten zu hohen Anteilen Wachse und Fettkomponenten, die nach dem Auftragen eine abdeckende Lipidschicht über den Lippen ausbilden.

Die Aufgabe dieser Schicht ist jedoch nicht vorderhand, die Lippenhaut vor dem Austrocknen zu schützen. Die Lipidschicht dient hier als auf den Lippen haftende Grundlage für die eingearbeiteten Pigmentstoffe; die Pigmente selbst können aus mancherlei Gründen nicht ohne eine solche Grundlage auf die Lippen aufgetragen werden.

Es war eine Aufgabe der vorliegenden Erfindung, Zubereitungen für die Lippenpflege zu entwickeln, welche sich durch hohe Pflegewirkung und leichte Handhabung auszeichnen, und welche darüberhinaus kosmetisch elegante Formulierungen darstellen würden.

Übliche, und sich gerade in neuerer Zeit immer weiter verbreitende kosmetische und dermatologische Zubereitungsformen sind Gele. Gele zeichnen sich durch halbfeste, oft fließfähige Konsistenz aus. Meistens sind Gele transparent oder tranzluzent, gelegentlich sind sie sogar klar. Die Gelstruktur wird durch ein inneres dreidimensionales molekulares Gerüst bewirkt, an welches durch elektrostatische Wechselwirkung die Molekeln einer äußeren Öl- oder Wasserphase locker gebunden sind. Im wesentlichen werden unterschieden: Oleogele, welche praktisch wasserfrei vorliegen, Hydrogele, welche praktisch fettfrei sind und Öl/Wasser-Gele, welche letztlich auf O/W- oder W/O-Emulsionen basieren, in welchen zusätzlich aber auch Merkmale einer Gelstruktur verwirklicht sind.

Übliche Grundstoffe des Standes der Technik sind Paraffinöle oder Paraffinwachse, die den Vorteil haben, daß paraffinhaltige Rezepturen, Emulsionen oder Oleogele, nicht sonderlich instabil gegenüber Temperaturschwankungen sind oder zumindest auf einfache Weise stabilisiert werden können. Paraffine bestehen aus verzweigten und unverzweigten gesättigten Kohlenwasserstoffen schwankender Kettenlänge.

Der Verzicht auf Paraffinöle- und Paraffinwachse kann von Vorteil sein. Beispielsweise ist zu begrüßen, wenn vorwiegend nachwachsende Rohstoffe oder auf solchen Rohstoffen basierende Substanzen verwendet werden, deren CO₂-Bilanz im Gegensatze zu mineralischen Lipidkomponenten neutral (oder wenigstens nahezu neutral) sind.

Auch war wünschenswert, das Spektrum der gängigen Formulierungsarten durch paraffinfreie Formulierungen zu erweitern.

Technisch betrachtet sind fast alle Lippenstifte wasserfreie Fettmischungen aus festen oder halbfesten Wachsen und flüssigen Ölen, wobei die hochgereinigten Paraffinöle und -wachse die Lippenstiftgrundmasse darstellen.

Nach dem idealen Anforderungsprofil sollen sich Lippenstifte glatt und ohne großen Reibungswiderstand auftragen lassen. Außerdem soll ein Lippenstift schon bei leichtem Andruck einen nicht schmierigen, stumpfen oder klebrigen, aber dennoch gut haftenden Fettfilm an die Lippen abgeben. Durch diesen Fettfilm sollen die Lippen dann glatt und geschmeidig gemacht werden.

Darüber hinaus muß ein Lippenstift auch noch die Anforderungen erfüllen, daß er bruchfest und temperaturbeständig sein muß und nicht ausölen darf.

Übliche Grundstoffe des Standes der Technik sind
(1) flüssige Öle (z.B. Paraffinöle, Ricinusöl, Isopropylmyristat)
(2) halbfeste Bestandteile (z.B. Vaseline, Lanolin)
(3) feste Bestandteile (z.B. Bienenwachs, Ceresin und Mikrokristalline Wachse bzw. Ozokerit)
(4) hochschmelzende Wachse (z.B. Carnaubawachs, Candelillawachs)

Lippenstifte des Standes der Technik mit einem Gehalt an Paraffinen und Bienenwachs sind in "Kosmetik, Entwicklung Herstellung und Anwendung kosmetischer Mittel", S. 105 Herausgeber: W.Umbach, Georg Thieme Verlag, Stuttgart - New York, 1988, beschrieben.

Der Stand der Technik hat aber eine Reihe von Nachteilen. So ist aus dem DBP 23 35 549 ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel herstellt, dieses mit einer kosmetischen Grundlage vermischt und dann einen Gehalt an Wasser in die Mischung. emulgiert.

Nach diesem Verfahren sind jedoch keine Stifte herzustellen, die über die gestellten universellen Anforderungen an einen kosmetischen Stift verfügen.

Ein weiterer Nachteil ist, daß Paraffinöle und -wachse bis zum gegenwärtigen Zeitpunkte unabdingbare Bestandteile für Lippenstifte waren. Obwohl es sich dabei um in guter Qualität erhältliche Rohstoffe handelt, und Stifte mit brauchbaren Eigenschaften mit ihrer Hilfe formuliert werden können, sind die Anwendungseigenschaften solcher kosmetischer Stifte jedoch begrenzt. Außerdem sind Paraffine wertvolle Grundstoffe, deren Vorkommen auf der Erde begrenzt sind. Die moderne Produktion geht in die Richtung nachwachsender Rohstoffe, also beispielsweise pflanzlicher Wachse oder Öle auf dem Gebiete der Kosmetik.

Es war jedoch bisher unmöglich, einen kosmetischen Stift auf der Basis der bekannten pflanzlichen Wachse, Fette oder Öle oder chemisch modifizierter pflanzlicher Wachse, Fette oder Öle zu konzipieren. Eine weiter Aufgabe der vorliegenden Erfindung war also, eine Grundlage für kosmetische Stifte, insbesondere Lippenstifte zu schaffen, welche auf mineralische Öle verzichten kann und stattdessen auf pflanzlichen oder gegebenenfalls tierischen Lipidkomponenten bzw. chemisch modifizierten Varianten davon basieren kann.

Insgesamt war also die Aufgabe des Standes der Technik, Formulierungen, zu entwikkeln, welche den Nachteilen des Standes der Technik in dieser Hinsicht abhelfen.

Erstaunlicherweise, und darin liegt die Lösung all dieser Aufgaben begründet, helfen kosmetische Formulierungen, welche im wesentlichen frei von gesättigten Kohlenwasserstoffen sind, umfassend
(a) eine Lipidphase enthaltend mindestens einen Glycerinmono-, -di-, und/oder -tricarbonsäureester verzweigter und/oder unverzweigter einbasiger Fettsäuren mit 18 - 36 Kohlenstoffatomen (= Mono-, Di- und/oder Triglyceride) sowie mindestens einen Ester gewählt aus der Gruppe der Substanzen mit folgender Struktur 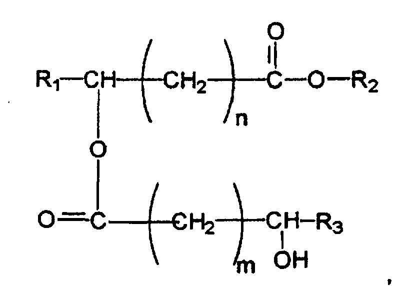 wobei
   R1 und R3 unabhängig voneinander einen verzweigten oder unverzweigten gesättigten Kohlenwasserstoffrest mit 3 - 30 Kohlenstoffatomen darstellen und R2 einen verzweigten oder unverzweigten gesättigten Kohlenwasserstoffrest mit 5 - 50 Kohlenstoffatomen darstellt und n und m unabhängig voneinander Werte von 5 bis 25 annehmen,
(d) femer gegebenenfalls enthaltend Wasser, weitere Lipide, Emulgatoren sowie übliche weitere Wirk-, Hilfs- und/oder Zusatzstoffe,
den Nachteilen des Standes der Technik ab.

Eine besondere Ausführungsform der vorliegenden Erfindung ist die Verwendung von kosmetischen Formulierungen, welche im wesentlichen frei von gesättigten Kohlenwasserstoffen sind, umfassend
(a) eine Lipidphase enthaltend mindestens einen Glycerinmono-, -di-, und/oder -tricarbonsäureester verzweigter und/oder unverzweigter einbasiger Fettsäuren mit 18 - 36 Kohlenstoffatomen (= Mono-, Di- und/oder Triglyceride) sowie mindestens einen Ester gewählt aus der Gruppe der Substanzen mit folgender Struktur 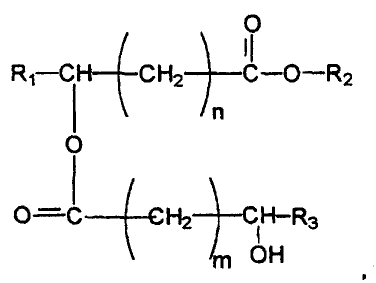 wobei
   R1 und R3 unabhängig voneinander einen verzweigten oder unverzweigten gesättigten Kohlenwasserstoffrest mit 3 - 30 Kohlenstoffatomen darstellen und R2 einen verzweigten oder unverzweigten gesättigten Kohlenwasserstoffrest mit 5 - 50 Kohlenstoffatomen darstellt und n und m unabhängig voneinander Werte von 5 bis 25 annehmen,
(d) ferner gegebenenfalls enthaltend weitere Lipide, Wasser, Emulgatoren sowie übliche weitere Wirk-, Hilfs- und/oder Zusatzstoffe,
als Lippenpflegeformulierung.

Die erfindungsgemäßen Zubereitungen können vorteilhaft als Oleogele, dann im wesentlichen wasserfrei, oder als W/O-Emulsionen vorliegen. Sie zeichnen sich durch hohe Temperaturstabilität, ausgezeichnete Haftfestigkeit und sehr guten Anwendungseigenschaften aus. Es ist auch möglich und vorteilhaft, die erfindungsgemäßen Zubereitungen in Form kosmetischer Stifte auszugestalten.

Das oder die Mono-, Di- und/oder Triglyceride liegt oder liegen erfindungsgemäß vorteilhaft in einem Gehalt von 0,5 - 10 Gew.-% vor, bezogen auf das Gesamtgewicht der Zubereitungen. Der oder die Ester gewählt aus der Gruppe der Substanzen mit folgender Struktur 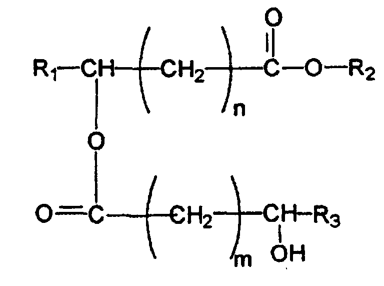 liegt oder liegen vorteilhaft in einem Gehalt von 15 - 25 Gew.-% vor, bezogen auf das Gesamtgewicht der Zubereitungen.

Bevorzugte Lipidkomponenten werden gewählt aus der Gruppe der Mono- Di- und Triglyceride sowie der Ester gewählt aus der Gruppe der Substanzen mit folgender Struktur 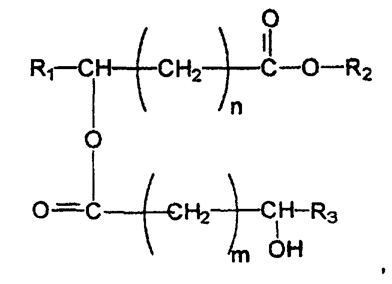 wobei
R1 und R3 unabhängig: voneinander einen verzweigten oder unverzweigten gesättigten Kohlenwasserstoffrest mit 5 - 10 Kohlenstoffatomen darstellen und R2 einen verzweigten oder unverzweigten gesättigten Kohlenwasserstoffrest mit 5 - 50 Kohlenstoffatomen darstellt und n und m unabhängig voneinander Werte von 5 bis 15 annehmen,

Bevorzugte Ester sind die C19-39-Alkyl-Hydroxystearoylstearate.

Die Merkmale der vorliegenden Erfindung sind vorteilhaft verwirklicht, wenn die Lipidphase zu 10 - 90 Gew.-%, bevorzugt 50 - 70 Gew.-%, aus flüssigen Lipiden, zu ≥ 0,5 Gew.-%, bevorzugt 5 - 30 Gew.-%, aus halbfesten Lipiden und zu 0,5 - 50 Gew.-%, bevorzugt 25 - 40 Gew.-%, aus festen Lipiden besteht, jeweils bezogen auf das Gesamtgewicht der Zubereitungen.

Wenn gewünscht ist, die erfindungsgemäßen Formulierungen als W/O-Emulsionen auszugestalten, kann der Wassergehalt vorteilhaft 0,1 - 15 Gew.-%, bevorzugt 3 - 5 Gew.-% betragen, jeweils bezogen auf das Gesamtgewicht der Zubereitungen.

Als fakultative, dennoch erfindungsgemäß vorteilhafte W/O-Emulgatoren können eingesetzt werden: Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Triglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Triglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Polyglycerin-2-polyhydroxystearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Behenylalkohol, Isobehenylalkohol, Selachylalkohol, Chimylalkohol.

Insbesondere, wenn die vorliegende Erfindung in kosmetischen Stiften verwirklicht wird, kann es vorteilhaft sein, als weitere Bestandteile Bienenwachs und/oder Ester aus gesättigten verzweigten oder unverzweigten aliphatischen Carbonsäuren mit 14 - 44 Kohlenstoffatomen und gesättigten verzweigten oder unverzweigten aliphatischen Alkoholen mit 14 - 44 Kohlenstoffatomen zu verwenden.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.Kombination.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofem Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Vorteilhaft enthalten die erfindungsgemäßen Zubereitungen mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, diedie Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon. 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtem zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Die erfindungsgemäßen kosmetischen und dermatologischen Zubereitungen können ferner kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Die nachfolgenden Beispiele sollen die vorliegende Erfindung verdeutlichen.

### Beispiele 1 - 6: Polare Lippengele mit hohem Fettanteil

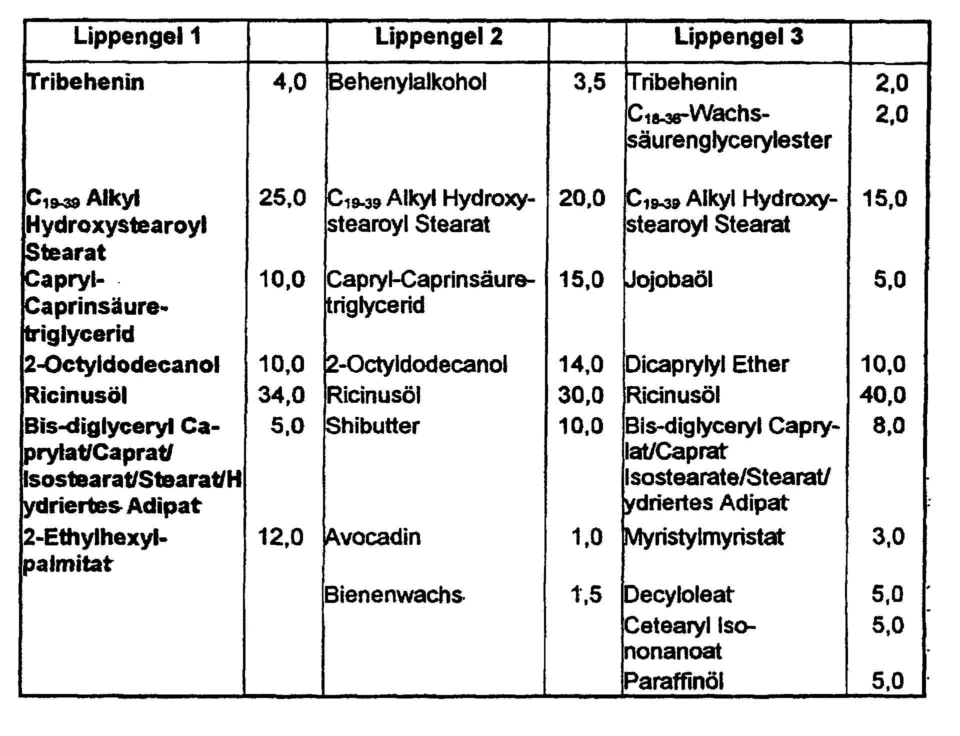

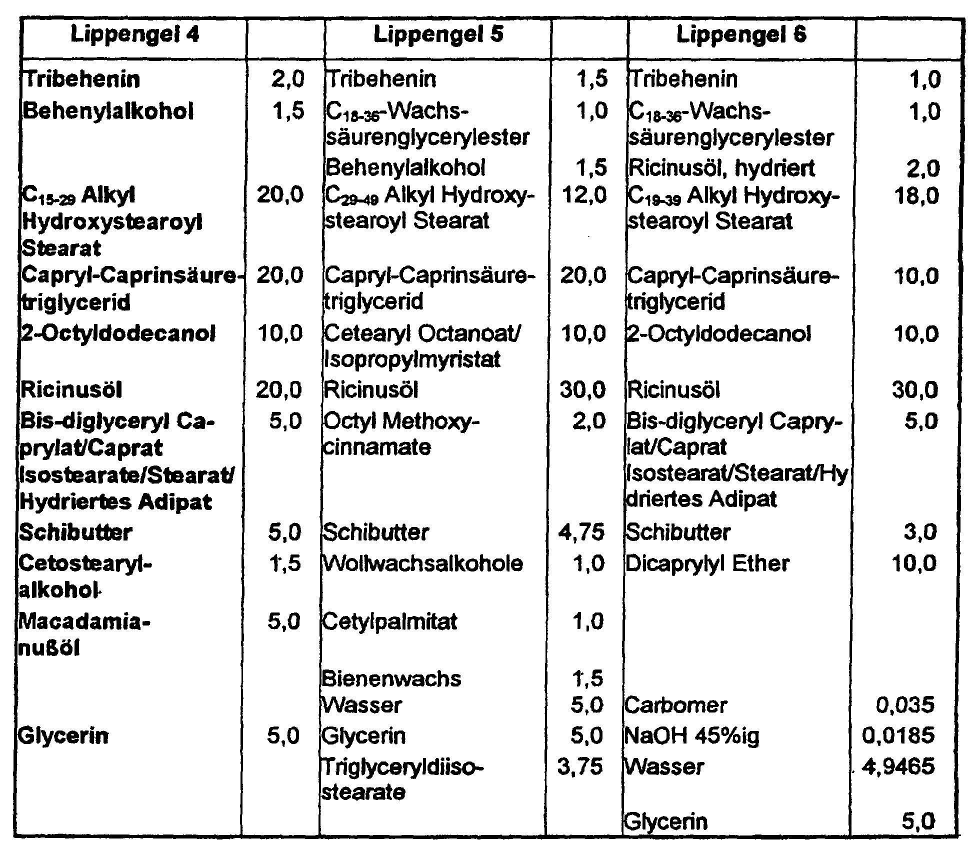

Die Fettkomponenten werden bei 90° - 95°C aufgeschmolzen. Getrennt davon wird die Wasserphase auf 70°C erhitzt und dann unter Rühren zur Fettphase gegeben. Die Masse wird unter Rühren abgekühlt.

### Beispiele 7 - 9: Stiftformulierungen

| **Stift 1 nicht erfindungsgemäß** | | **Stift 2** | | **Stift 3** | |
|---|---|---|---|---|---|
| **mittelpolar** | | **mittelpolar** | | **polar** | |
| Tribehenin | 1,0% | Tribehenin | 1,0% | Tribehenin | 2,5% |
| C₁₉₋₃₉ Alkyl Hydroxystearoyl Stearat | 1,0% | C₁₉₋₃₉ Alkyl Hydroxystearoyl Stearat | 0,5% | C₁₉₋₃₉ Alkyl Hydroxystearoyl Stearat | 2,5% |
| Carnaubawachs | 1,3% | Carnaubawachs | 1,6% | Carnaubawachs | 2,5% |
| Schibutter | 1,5% | C₁₈₋₃₆-Wachssäurenglycerylester | 1,0% | Schibutter | 2,5% |
| Myristylmyristat | 9,0% | Myristylmyristat | 10,0% | Jojobawachs hydriert | 4,5% |
| Ceresin | 12,0% | Ceresin | 22,4% | Candelillawachs | 6,0% |
| Cetostearylalkohol | 2,5% | Cetostearylalkohol | 2,5% | Bis-diglyceryl Caprylat/Caprat/Isostearat/Stearat/Hydriertes Adipat | 8,0% |
| Hydierte Kokosfettsäureglyceride | 5,0% | | | Hydierte Kokosfettsäureglyceride | 3,0% |
| Ozokerit | 6,0% | | | Bienenwachs | 5,0% |
| Capryl-Caprinsäure-Triglycerid | 26,7% | Capryl-Caprinsäure-Triglycerid | 22,0% | Capryl-Caprinsäure-Triglycerid | 10,0% |
| Ricinusöl | 12,0% | Ricinusöl | 12,0% | Ricinusöl | 13,5% |
| Dicaprylyl Ether | 22,0% | 2-Octyldodecanol | 27,0% | Dicapryl Ether | 20,0% |
| | | | | Jojobaöl | 4,0% |
| | | | | Avocadoöl | 4,0% |
| | | | | Macadamianußöl | 4,0% |
| | | | | Squalan | 4,0% |
| | | | | Mango-Kern-Fett | 2,0% |
| | | | | Cholesteryl/Behenyl/Octydodecyl Lauroyl Glutamat | 2,0% |

Die Fettkomponenten werden bei 90° - 95°C aufgeschmolzen. Die Masse kühlt unter Rühren auf 70°C ab und wird dann vergossen.

### Beispiele 10 - 14: W/O-Emulsionen

| **Emulsion 1** | | **Emulsion 2** | | **Emulsion 3** | | **Emulsion 4** | |
|---|---|---|---|---|---|---|---|
| **polare Fettpha- se** | | **mittelpolar** | | **mittelpolar** | | **mittelpolar** | |
| Tribehenin | 1,0% | Tribehenin | 1,5% | Tribehenin | 0,75% | Tribehenin | 0,5% |
| C₁₉₋₃₉ AlkylHydroxystearoylstearat | 4,0% | C₁₉₋₃₉ AlkylHydroxystearoylstearat | 2,0% | C₁₉₋₃₉ AlkylHydroxystearoylstearat | 3,0% | C₁₉₋₃₉ AlkylHydroxystearoylstearat | 4,0% |
| Triglyceryldiisostearate | 3,0% | Triglyceryldiisostearate | 2,4% | Triglyceryldiisostearate | 2,4% | Glycerinsorbitan fettsäureester | 3,0% |
| Polyglyceryl-2 Polyhydroxysteareat | 3,0% | DicocoylPentaerythrityl-Distearyl Citrat | 1,2% | DicocoylPentaerythrityl-Distearyl Citrat | 1,2% | Glyceryl Isostearat | 2,0% |
| Isopropylstearat | 4,0% | Squalan | 10,0 % | Squalan | 12,0% | Polydecen | 10,0 % |
| Polyisobuten | 2,0% | Cetearyl Octanoat/Isopropyl myristate | 5,0% | Decyloleat | 5,0% | Octyl Cocoat | 5,5% |
| Capryl-Caprinsäure-Triglycerid | 3,5% | Cetearyl Isononaoate | 5,0% | Schibutter | 6,5% | Cetearyl Isononaoat | 3,5% |
| Macadamianußöl | 1,5% | | | | | | |
| Octyl Cocoate | 5,6% | | | | | | |
| Phenoxyethanol | 0,4% | Phenoxyethanol | 0,4% | Phenoxyethanol | 0,4% | Phenoxyethanol | 0,4% |
| Methylparaben | 0,18 % | Methylparaben | 0,18 % | Methylparaben | 0,18% | Methylparaben | 0,18 % |
| Propylparaben | 0,02 % | Propylparaben | 0,02 % | Propylparaben | 0,02% | Propylparaben | 0,02 % |
| Glycerin | 3,0% | Glycerin | 3,0% | Glycerin | 3,0% | Glycerin | 3,0% |
| MgSO₄ | 0,7% | MgSO₄ | 0,7% | MgSO₄ | 0,7% | MgSO₄ | 0,7% |
| Wasser | 68,1 % | Wasser | 68,7 % | Wasser | 64,85 % | Wasser | 67,2 % |

Die Fettphase wird auf 85°C erhitzt bis die festen Bestandteile geschmolzen sind. Die Wasserphase wird getrennt auf 75°C erwärmt und unter Rühren zur Fettphase gegen. Die Emulsion kühlt unter Rühren auf 35°C ab und wird dann homogenisiert.

### Beispiel 14: Kälteschutzcreme

| | |
|---|---|
| Tribehenin | 3,5% |
| C₁₉₋₃₉ Alkyl Hydroxystearoyl Stearate | 20,0% |
| Bis-diglyceryl Caprylate/Caprate/Isostearate/Stearate/Hydrogenated Adipate | 3,5% |
| Capryl-Caprinsäure-Triglycerid | 8,0% |
| 2-Octyldodecanol | 8,0% |
| Dicaprylyl Ether | 6,0% |
| 2-Ethylhexylpalmitat | 6,0% |
| Ricinusöl | 32,0% |
| Avocadin | 2,5% |
| Bisabolol | 0,5% |
| Zinkoxid | 5,0% |
| Talkum | 5,0% |

### Beispiel 15: Baby Wundcreme

| | |
|---|---|
| Tribehenin | 3,0% |
| C₁₉₋₃₉ Alkyl Hydroxystearoyl Stearate | 18,5% |
| Wollwachsalkohole | 1,0% |
| Glyceryl Lanolate | 2,0% |
| Sheabutter | 5,0% |
| Capryl-Caprinsäure-Triglycerid | 10,0% |
| 2-Octyldodecanol | 10,0% |
| Ricinusöl | 23,5% |
| Bisabolol | 1,0% |
| Titandioxid | 3,0% |
| Zinkoxid | 5,0% |
| Kaolin | 13,0% |
| Talkum | 5,0% |

4) und 5) Die Fettkomponente werden bei 90° - 95° geschmolzen, anschließend werden die Pigmente untergerührt und mit dem Turrax dispergiert.

## Patentansprüche

1. Kosmetische Zubereitungen, welche im wesentlichen frei von gesättigten Kohlenwasserstoffen sind, umfassend
(a) eine Lipidphase enthaltend mindestens einen Glycerinmono-, -di-, und/oder -tricarbonsäureester verzweigter und/oder unverzweigter einbasiger Fettsäuren mit 18 - 36 Kohlenstoffatomen (= Mono-, Di- und/oder Triglyceride) sowie mindestens einen Ester gewählt aus der Gruppe der Substanzen mit folgender Struktur 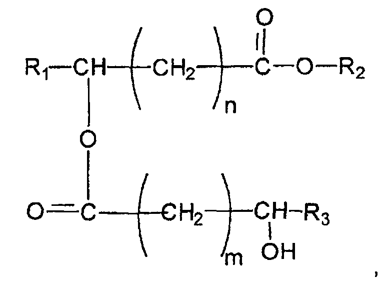 wobei
R1 und R3 unabhängig voneinander einen verzweigten oder unverzweigten gesättigten Kohlenwasserstoffrest mit 3 - 30 Kohlenstoffatomen darstellen und R2 einen verzweigten oder unverzweigten gesättigten Kohlenwasserstoffrest mit 5 - 50 Kohlenstoffatomen darstellt und n und m unabhängig voneinander Werte von 5 bis 25 annehmen,
(b) ferner gegebenenfalls enthaltend weitere Lipide, Wasser, Emulgatoren sowie übliche weitere Wirk-, Hilfs- und/oder Zusatzstoffe.

2. Zubereitungen nach Anspruch 1, **dadurch gekennzeichnet, daß** das oder die Mono-, Di- und/oder Triglyceride in einem Gehalt von 2,5 - 5 Gew.-% vor liegt oder liegen, jeweils bezogen auf das Gesamtgewicht der Zubereitungen.

3. Zubereitungen nach Anspruch 1, **dadurch gekennzeichnet, daß** der oder die Ester gewählt aus der Gruppe der Substanzen mit folgender Struktur 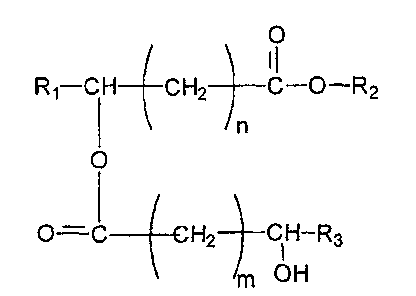 in einem Gehalt von 15 - 25 Gew.-% vorliegt oder liegen, jeweils bezogen auf das Gesamtgewicht der Zubereitungen.

4. Zubereitungen nach Anspruch 1, **dadurch gekennzeichnet, daß** die Lipidphase zu 10 - 90 Gew.-%, bevorzugt 50 - 70 Gew.-%; aus flüssigen Lipiden, zu ≥ 0,5 Gew.-%, bevorzugt 5 - 30 Gew.-%, aus halbfesten Lipiden und zu 0,5 - 50 Gew.-%, bevorzugt 25 - 40 Gew.-%, aus festen Lipiden besteht, jeweils bezogen auf das Gesamtgewicht der Zubereitungen.

5. Zubereitungen nach Anspruch 1, **dadurch gekennzeichnet, daß** der Wassergehalt 0,1 - 15 Gew.-%, bevorzugt 3 - 5 Gew.-% beträgt, jeweils bezogen auf das Gesamtgewicht der Zubereitungen.

## Claims

1. Cosmetic formulations which are essentially free from saturated hydrocarbons and comprise
(a) a lipid phase comprising at least one glycerol mono-, di- and/or tricarboxylic acid ester of branched and/or unbranched monobasic fatty acids having 18-36 carbon atoms (= mono-, di- and/or triglycerides) and at least one ester chosen from the group consisting of substances having the following structure 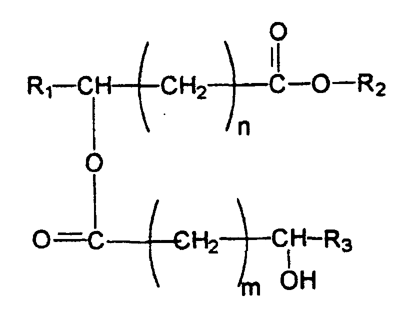 wherein
R1 and R3 independently of one another are a branched or unbranched saturated hydrocarbon radical having 3-30 carbon atoms and R2 is a branched or unbranched saturated hydrocarbon radical having 5-50 carbon atoms, and n and m independently of one another assume values from 5 to 25,
(b) and furthermore, if appropriate, comprise further lipids, water, emulsifiers and customary further active compounds, auxiliaries and/or additives.

2. Formulations according to Claim 1, **characterized in that** the mono-, di- and/or triglyceride or - glycerides is or are present in a content of 2.5 - 5% by weight, in each case based on the total weight of the formulations.

3. Formulations according to Claim 1, **characterized in that** the ester or esters chosen from the group of substances having the following structure 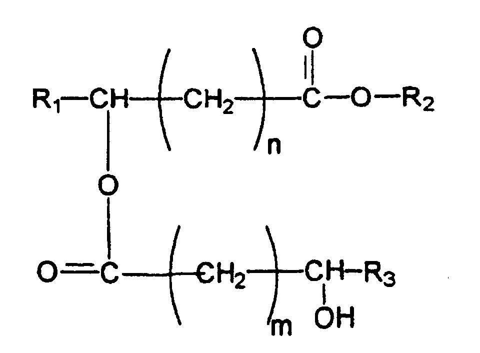 is or are present in a content of 15 - 25% by weight, in each case based on the total weight of the formulations.

4. Formulations according to Claim 1, **characterized in that** the lipid phase comprises liquid lipids to the extent of 10 - 90% by weight, preferably 50 - 70% by weight, semi-solid lipids to the extent of ≥ 0.5% by weight, preferably 5 - 30% by weight, and solid lipids to the extent of 0.5 - 50% by weight, preferably 25 - 40% by weight, in each case based on the total weight of the formulations.

5. Formulations according to Claim 1, **characterized in that** the water content is 0.1 - 15% by weight, preferably 3 - 5% by weight, in each case based on the total weight of the formulations.

## Revendications

1. Formulations cosmétiques essentiellement exemptes d'hydrocarbures saturés, comprenant
a) une phase lipidique comprenant au moins un ester mono-, di- et/ou tricarboxylique du glycérol et d'acides gras monobasiques ramifiés et/ou non ramifiés ayant de 18 à 36 atomes de carbone (= mono-, di- et/ou triglycérides) et au moins un ester choisi parmi le groupe des substances de structure suivante 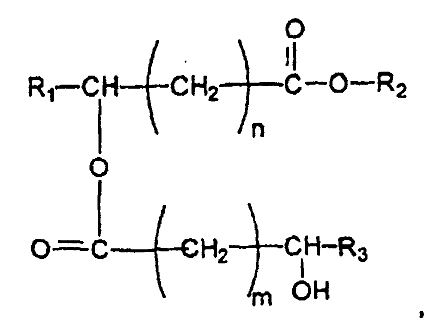 dans laquelle
R1 et R3 représentent, indépendamment l'un de l'autre, un radical hydrocarboné saturé ramifié ou non ramifié ayant de 3 à 30 atomes de carbone et R2 représente un radical hydrocarboné saturé ramifié ou non ramifié ayant de 5 à 50 atomes de carbone, et n et m prennent, indépendamment l'un de l'autre, des valeurs de 5 à 25,
b) et comprenant en outre éventuellement d'autres lipides, de l'eau, des émulsifiants ainsi que d'autres ingrédients actifs, auxiliaires' et/ou additifs habituels.

2. Formulations selon la revendication 1, **caractérisées en ce que** le ou les mono-, di- et/ou triglycéride(s) est (sont) présent(s) en une teneur de 2,5 à 5 % en poids, dans chaque cas par rapport au poids total des formulations.

3. Formulations selon la revendication 1, **caractérisées en ce que** le ou les ester(s) choisi(s) parmi le groupe des substances de structure suivante 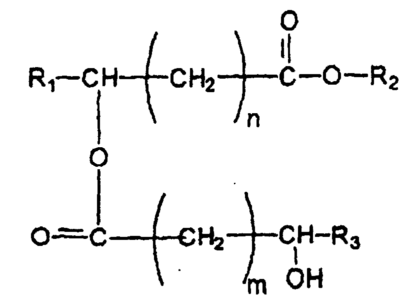 est (sont) présent(s) en une teneur de 15 à 25 % en poids, dans chaque cas par rapport au poids total des formulations.

4. Formulations selon la revendication 1, **caractérisées en ce que** la phase lipidique est constituée de 10 à 90 % en poids, de préférence de 50 à 70 % en poids de lipides liquides, de plus de 0,5 % en poids, de préférence de 5 à 30 % en poids de lipides semi-liquides et de 0,5 à 50 % en poids, de préférence de 25 à 40 % en poids de lipides solides, dans chaque cas par rapport au poids total des formulations.

5. Formulations selon la revendication 1, **caractérisées en ce que** la teneur en eau est de 0,1 à 15 % en poids, de préférence de 3 à 5 % en poids, dans chaque cas par rapport au poids total des formulations.
